# EUROPEAN PATENT APPLICATION

(11) **EP 4 343 780 A1**
(43) Date of publication of application: **27.03.2024**
(21) Application number: 23196980.9
(22) Date of filing: 12.09.2023
(51) Int. Cl.: G16H 30/40, G16H 50/20, G06F 3/04842, G06T 7/00

(54) **INFORMATION PROCESSING APPARATUS, INFORMATION PROCESSING METHOD, AND INFORMATION PROCESSING PROGRAM**

(30) Priority: 21.09.2022 JP 2022150754
(71) Applicant: FUJIFILM Corporation, Tokyo 106-8620 (JP)
(72) Inventor: HATSUTANI, Taro, Tokyo, 106-8620 (JP); ICHINOSE, Akimichi, Tokyo, 106-8620 (JP)
(74) Representative: Klunker IP Patentanwälte PartG mbB

(57) **Abstract**

Provided are an information processing apparatus, an information processing method, and an information processing program capable of supporting interpretation of medical images.

An information processing apparatus includes at least one processor, and the processor is configured to: acquire a medical image including a plurality of lesion regions; classify a high or low degree of interest of at least one first lesion region among the plurality of lesion regions; specify at least one second lesion region similar to the first lesion region among the plurality of lesion regions in a case where the degree of interest is low; and make a display method of the first lesion region and the second lesion region classified as having a low degree of interest different from a display method of other lesion regions in a case where the plurality of lesion regions are highlighted and the medical image is displayed on a display.

## Description

### BACKGROUND OF THE INVENTION

### 1. Field of the Invention

The present disclosure relates to an information processing apparatus, an information processing method, and an information processing program.

### 2. Description of the Related Art

In the related art, image diagnosis is performed using medical images obtained by imaging apparatuses such as computed tomography (CT) apparatuses and magnetic resonance imaging (MRI) apparatuses. In addition, medical images are analyzed via computer aided detection/diagnosis (CAD) using a discriminator in which learning is performed by deep learning or the like, and regions of interest including structures, lesions, and the like included in the medical images are detected and/or diagnosed. The medical images and analysis results via CAD are transmitted to a terminal of a healthcare professional such as a radiologist who interprets the medical images. The healthcare professional such as a radiologist interprets the medical image by referring to the medical image and analysis result using his or her own terminal and creates an interpretation report.

In addition, various methods for supporting the interpretation of medical images have been proposed. For example, JP2019-153250A discloses a technique for creating an interpretation report based on a keyword input by a radiologist and an analysis result of a medical image. In the technique disclosed in JP2019-153250A, a sentence to be included in the interpretation report is created by using a recurrent neural network trained to generate a sentence from input characters.

Further, for example, JP2009-082442A discloses a technique for enabling efficient reference to information related to a lesion part by acquiring information on a lesion candidate based on a medical image and outputting data in which a medical importance of the lesion candidate is higher than a threshold value.

### SUMMARY OF THE INVENTION

Among the lesions detected from the medical image, there is a case where a lesion having a high degree of interest to a doctor and a lesion having a low degree of interest to a doctor are mixed. For example, a lesion that does not require treatment, follow-up observation, and the like is of a low clinical value and is of a lower degree of interest to a doctor than a lesion that requires treatment, follow-up observation, and the like. In such a case, presenting all the detected lesions including the lesion having a low degree of interest may hinder interpretation.

Further, a plurality of similar lesions may be included in the lesions detected from the medical image, and it is assumed that the degree of interest thereof is the same. Therefore, there is a demand for a technique capable of more efficiently supporting interpretation of a medical image by changing a display method of each lesion according to a degree of interest while utilizing the degree of interest of similar lesions.

The present disclosure provides an information processing apparatus, an information processing method, and an information processing program capable of supporting interpretation of medical images.

According to a first aspect of the present disclosure, there is provided an information processing apparatus comprising at least one processor, in which the processor is configured to: acquire a medical image including a plurality of lesion regions; classify a high or low degree of interest of at least one first lesion region among the plurality of lesion regions; specify at least one second lesion region similar to the first lesion region among the plurality of lesion regions in a case where the degree of interest is low; and make a display method of the first lesion region and the second lesion region classified as having a low degree of interest different from a display method of other lesion regions in a case where the plurality of lesion regions are highlighted and the medical image is displayed on a display.

According to a second aspect of the present disclosure, in the first aspect, the processor may be configured to display the medical image on the display such that the first lesion region and the second lesion region classified as having a low degree of interest are not emphasized more than the other lesion regions.

According to a third aspect of the present disclosure, in the first aspect or the second aspect, the processor may be configured to specify the second lesion region based on an image feature of the medical image.

According to a fourth aspect of the present disclosure, in any one of the first to third aspects, the processor may be configured to receive an input of the high or low degree of interest.

According to a fifth aspect of the present disclosure, in any one of the first to fourth aspects, the processor may be configured to acquire a predetermined degree of interest for a lesion region similar to the first lesion region as a degree of interest of the first lesion region.

According to a sixth aspect of the present disclosure, in any one of the first to fifth aspects, the processor may be configured to estimate a degree of interest of the first lesion region based on whether or not there is a description of a lesion region similar to the first lesion region in an interpretation report describing another medical image including the lesion region.

According to a seventh aspect of the present disclosure, in the sixth aspect, the processor may be configured to estimate that a degree of interest of the first lesion region is low in a case where there is no description of the lesion region similar to the first lesion region in the interpretation report describing another medical image including the lesion region.

According to an eighth aspect of the present disclosure, in any one of the first to seventh aspects, the processor may be configured to specify, as the second lesion region, a lesion region similar to the first lesion region among lesion regions in which a distance from the first lesion region is within a predetermined range.

According to a ninth aspect of the present disclosure, there is provided an information processing method comprising: acquiring a medical image including a plurality of lesion regions; classifying a high or low degree of interest of at least one first lesion region among the plurality of lesion regions; specifying at least one second lesion region similar to the first lesion region among the plurality of lesion regions in a case where the degree of interest is low; and making a display method of the first lesion region and the second lesion region classified as having a low degree of interest different from a display method of other lesion regions in a case where the plurality of lesion regions are highlighted and the medical image is displayed on a display.

According to a tenth aspect of the present disclosure, there is provided an information processing program for causing a computer to execute a process comprising: acquiring a medical image including a plurality of lesion regions; classifying a high or low degree of interest of at least one first lesion region among the plurality of lesion regions; specifying at least one second lesion region similar to the first lesion region among the plurality of lesion regions in a case where the degree of interest is low; and making a display method of the first lesion region and the second lesion region classified as having a low degree of interest different from a display method of other lesion regions in a case where the plurality of lesion regions are highlighted and the medical image is displayed on a display.

With the information processing apparatus, the information processing method, and the information processing program according to the aspects of the present disclosure, it is possible to support the interpretation of medical images.

### BRIEF DESCRIPTION OF THE DRAWINGS

Fig. 1 is a diagram showing an example of a schematic configuration of an information processing system.
Fig. 2 is a diagram showing an example of a medical image.
Fig. 3 is a diagram showing an example of a medical image.
Fig. 4 is a block diagram showing an example of a hardware configuration of an information processing apparatus.
Fig. 5 is a block diagram showing an example of a functional configuration of the information processing apparatus.
Fig. 6 is a diagram showing an example of a screen displayed on a display.
Fig. 7 is a diagram showing an example of a screen displayed on a display.
Fig. 8 is a diagram showing an example of a screen displayed on a display.
Fig. 9 is a diagram showing an example of a screen displayed on a display.
Fig. 10 is a flowchart showing an example of information processing.

### DESCRIPTION OF THE PREFERRED EMBODIMENTS

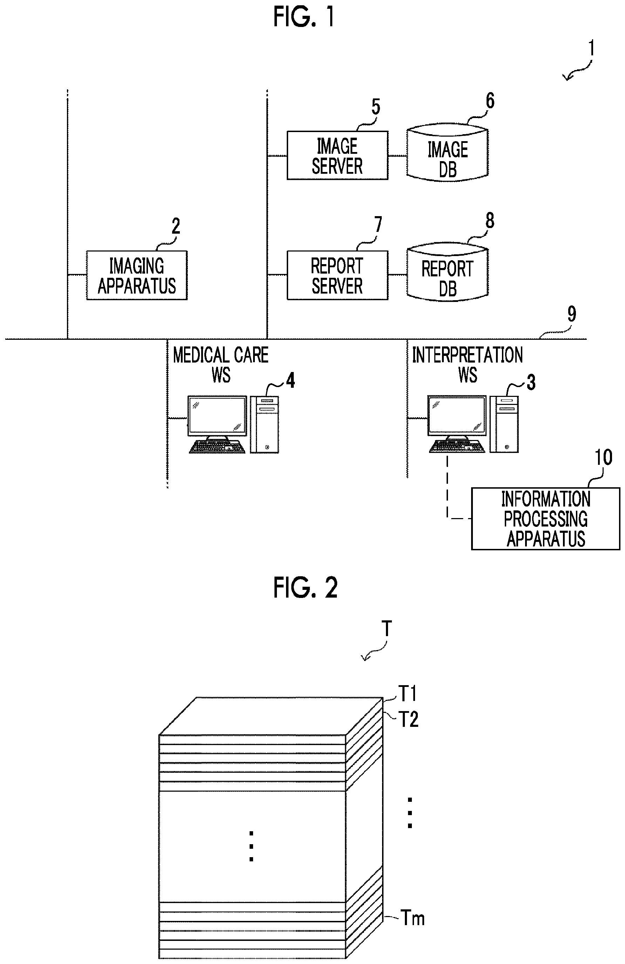

Hereinafter, an embodiment of the present disclosure will be described with reference to the drawings. First, a configuration of an information processing system 1 to which an information processing apparatus 10 of the present disclosure is applied will be described. Fig. 1 is a diagram showing a schematic configuration of the information processing system 1. The information processing system 1 shown in Fig. 1 performs imaging of an examination target part of a subject and storing of a medical image acquired by the imaging based on an examination order from a doctor in a medical department using a known ordering system. In addition, the information processing system 1 performs an interpretation work of a medical image and creation of an interpretation report by a radiologist and viewing of the interpretation report by a doctor of a medical department that is a request source.

As shown in Fig. 1, the information processing system 1 includes an imaging apparatus 2, an interpretation work station (WS) 3 that is an interpretation terminal, a medical care WS 4, an image server 5, an image database (DB) 6, a report server 7, and a report DB 8. The imaging apparatus 2, the interpretation WS 3, the medical care WS 4, the image server 5, the image DB 6, the report server 7, and the report DB 8 are connected to each other via a wired or wireless network 9 in a communicable state.

Each apparatus is a computer on which an application program for causing each apparatus to function as a component of the information processing system 1 is installed. The application program may be recorded on, for example, a recording medium, such as a digital versatile disc read only memory (DVD-ROM) or a compact disc read only memory (CD-ROM), and distributed, and be installed on the computer from the recording medium. In addition, the application program may be stored in, for example, a storage apparatus of a server computer connected to the network 9 or in a network storage in a state in which it can be accessed from the outside, and be downloaded and installed on the computer in response to a request.

The imaging apparatus 2 is an apparatus (modality) that generates a medical image showing a diagnosis target part of the subject by imaging the diagnosis target part. Examples of the imaging apparatus 2 include a simple X-ray imaging apparatus, a computed tomography (CT) apparatus, a magnetic resonance imaging (MRI) apparatus, a positron emission tomography (PET) apparatus, an ultrasound diagnostic apparatus, an endoscope, a fundus camera, and the like. The medical image generated by the imaging apparatus 2 is transmitted to the image server 5 and is saved in the image DB 6.

Fig. 2 is a diagram schematically showing an example of a medical image acquired by the imaging apparatus 2. A medical image T shown in Fig. 2 is, for example, a CT image consisting of a plurality of tomographic images T1 to Tm (m is 2 or more) representing tomographic planes from the head to the lumbar region of one subject (human body).

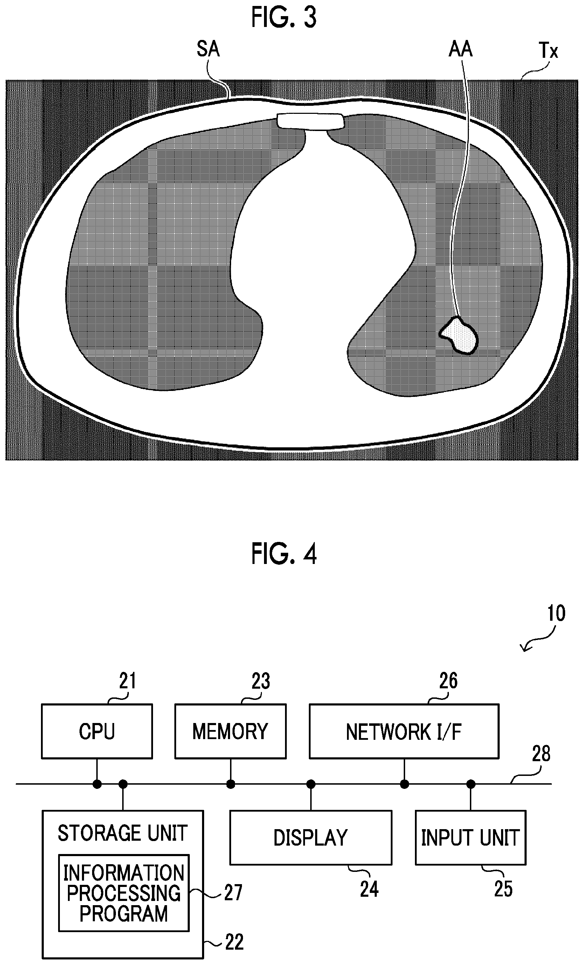

Fig. 3 is a diagram schematically showing an example of one tomographic image Tx out of the plurality of tomographic images T1 to Tm. The tomographic image Tx shown in Fig. 3 represents a tomographic plane including a lung. Each of the tomographic images T1 to Tm may include a region SA of a structure showing various organs and viscera of the human body (for example, lungs, livers, and the like), various tissues constituting various organs and viscera (for example, blood vessels, nerves, muscles, and the like), and the like. In addition, each tomographic image may include a lesion region AA such as, for example, nodules, tumors, injuries, defects, and inflammation. In the tomographic image Tx shown in Fig. 3, the lung region is the region SA of the structure, and the nodule region is the lesion region AA. A single tomographic image may include regions SA of a plurality of structures and/or lesion regions AA. Hereinafter, at least one of the region SA of the structure included in the medical image or the lesion region AA included in the medical image will be referred to as a "region of interest".

The interpretation WS 3 is a computer used by, for example, a healthcare professional such as a radiologist of a radiology department to interpret a medical image and to create an interpretation report, and encompasses an information processing apparatus 10 according to the present embodiment. In the interpretation WS 3, a viewing request for a medical image to the image server 5, various image processing for the medical image received from the image server 5, display of the medical image, and input reception of a sentence regarding the medical image are performed. In the interpretation WS 3, an analysis process for medical images, support for creating an interpretation report based on the analysis result, a registration request and a viewing request for the interpretation report to the report server 7, and display of the interpretation report received from the report server 7 are performed. The above processes are performed by the interpretation WS 3 executing software programs for respective processes.

The medical care WS 4 is a computer used by, for example, a healthcare professional such as a doctor in a medical department to observe a medical image in detail, view an interpretation report, create an electronic medical record, and the like, and includes a processing apparatus, a display apparatus such as a display, and an input apparatus such as a keyboard and a mouse. In the medical care WS 4, a viewing request for the medical image to the image server 5, display of the medical image received from the image server 5, a viewing request for the interpretation report to the report server 7, and display of the interpretation report received from the report server 7 are performed. The above processes are performed by the medical care WS 4 executing software programs for respective processes.

The image server 5 is a general-purpose computer on which a software program that provides a function of a database management system (DBMS) is installed. The image server 5 is connected to the image DB 6. The connection form between the image server 5 and the image DB 6 is not particularly limited, and may be a form connected by a data bus, or a form connected to each other via a network such as a network attached storage (NAS) and a storage area network (SAN).

The image DB 6 is realized by, for example, a storage medium such as a hard disk drive (HDD), a solid state drive (SSD), and a flash memory. In the image DB 6, the medical image acquired by the imaging apparatus 2 and accessory information attached to the medical image are registered in association with each other.

The accessory information may include, for example, identification information such as an image identification (ID) for identifying a medical image, a tomographic ID assigned to each tomographic image included in the medical image, a subject ID for identifying a subject, and an examination ID for identifying an examination. In addition, the accessory information may include, for example, information related to imaging such as an imaging method, an imaging condition, an imaging purpose, and an imaging date and time related to imaging of a medical image. The "imaging method" and "imaging condition" are, for example, a type of the imaging apparatus 2, an imaging part, an imaging protocol, an imaging sequence, an imaging method, the presence or absence of use of a contrast medium, a slice thickness in tomographic imaging, and the like. In addition, the accessory information may include information related to the subject such as the name, date of birth, age, and gender of the subject.

In a case where the image server 5 receives a request to register a medical image from the imaging apparatus 2, the image server 5 prepares the medical image in a format for a database and registers the medical image in the image DB 6. In addition, in a case where the viewing request from the interpretation WS 3 and the medical care WS 4 is received, the image server 5 searches for a medical image registered in the image DB 6 and transmits the searched for medical image to the interpretation WS 3 and to the medical care WS 4 that are viewing request sources.

The report server 7 is a general-purpose computer on which a software program that provides a function of a database management system is installed. The report server 7 is connected to the report DB 8. The connection form between the report server 7 and the report DB 8 is not particularly limited, and may be a form connected by a data bus or a form connected via a network such as a NAS and a SAN.

The report DB 8 is realized by, for example, a storage medium such as an HDD, an SSD, and a flash memory. In the report DB 8, an interpretation report created in the interpretation WS 3 is registered. In addition, the report DB 8 may store findings information regarding the medical image. Findings information includes, for example, information obtained by the interpretation WS 3 through image analysis of a medical image using a computer aided detection/diagnosis (CAD) technology, an artificial intelligence (Al) technology, or the like, and information or the like input by a user after interpreting a medical image.

For example, findings information includes information indicating various findings such as a name (type), a property, a position, a measurement value, and an estimated disease name of a region of interest included in the medical image. Examples of names (types) include the names of structures such as "lung" and "liver", and the names of lesions such as "nodule". The property mainly means the features of the lesion. For example, in the case of a lung nodule, findings indicating absorption values such as "solid type" and "frosted glass type", margin shapes such as "clear/unclear", "smooth/irregular", "spicula", "lobulation", and "serration", and an overall shape such as "round shape" and "irregular shape" can be mentioned. In addition, for example, there are findings regarding the relationship with surrounding tissues such as "pleural contact" and "pleural invagination", and the presence or absence of contrast, washout, and the like.

The position means an anatomical position, a position in a medical image, and a relative positional relationship with other regions of interest such as "inside", "margin", and "periphery". The anatomical position may be indicated by an organ name such as "lung" and "liver", and may be expressed in terms of subdivided lungs such as "right lung", "upper lobe", and apical segment ("S 1 "). The measurement value is a value that can be quantitatively measured from a medical image, and is, for example, at least one of a size or a signal value of a region of interest. The size is represented by, for example, a major axis, a minor axis, an area, a volume, or the like of a region of interest. The signal value is represented by, for example, a pixel value in a region of interest, a CT value in units of HU, or the like. The estimated disease name is an evaluation result estimated based on the lesion, and, for example, the disease name such as "cancer" and "inflammation" and the evaluation result such as "negative/positive", "benign/malignant", and "mild/severe" regarding disease names and properties can be mentioned.

Further, the report DB 8 may store a high or low degree of interest (details will be described later) determined for the lesion region included in the medical image.

Further, in a case where the report server 7 receives a request to register the interpretation report from the interpretation WS 3, the report server 7 prepares the interpretation report in a format for a database and registers the interpretation report in the report DB 8. Further, in a case where the report server 7 receives the viewing request for the interpretation report from the interpretation WS 3 and the medical care WS 4, the report server 7 searches for the interpretation report registered in the report DB 8, and transmits the searched for interpretation report to the interpretation WS 3 and to the medical care WS 4 that are viewing request sources.

The network 9 is, for example, a network such as a local area network (LAN) and a wide area network (WAN). The imaging apparatus 2, the interpretation WS 3, the medical care WS 4, the image server 5, the image DB 6, the report server 7, and the report DB 8 included in the information processing system 1 may be disposed in the same medical institution, or may be disposed in different medical institutions or the like. Further, the number of each apparatus of the imaging apparatus 2, the interpretation WS 3, the medical care WS 4, the image server 5, the image DB 6, the report server 7, and the report DB 8 is not limited to the number shown in Fig. 1, and each apparatus may be composed of a plurality of apparatuses having the same functions.

Incidentally, among the lesions detected from the medical image, there is a case where a lesion having a high degree of interest to a doctor and a lesion having a low degree of interest to a doctor are mixed. For example, a lesion that does not require treatment, follow-up observation, and the like is of a low clinical value and is of a lower degree of interest to a doctor than a lesion that requires treatment, follow-up observation, and the like. Further, a plurality of similar lesions may be included in the lesions detected from the medical image, and it is assumed that the degree of interest thereof is the same.

Therefore, the information processing apparatus 10 according to the present embodiment has a function of more efficiently supporting interpretation of a medical image by changing a display method of each lesion according to a degree of interest while utilizing the degree of interest of similar lesions. The information processing apparatus 10 will be described below. As described above, the information processing apparatus 10 is encompassed in the interpretation WS 3.

First, with reference to Fig. 4, an example of a hardware configuration of the information processing apparatus 10 according to the present embodiment will be described. As shown in Fig. 4, the information processing apparatus 10 includes a central processing unit (CPU) 21, a non-volatile storage unit 22, and a memory 23 as a temporary storage area. Further, the information processing apparatus 10 includes a display 24 such as a liquid crystal display, an input unit 25 such as a keyboard and a mouse, and a network interface (I/F) 26. The network I/F 26 is connected to the network 9 and performs wired and/or wireless communication. The CPU 21, the storage unit 22, the memory 23, the display 24, the input unit 25, and the network I/F 26 are connected to each other via a bus 28 such as a system bus and a control bus so that various types of information can be exchanged.

The storage unit 22 is realized by, for example, a storage medium such as an HDD, an SSD, and a flash memory. An information processing program 27 in the information processing apparatus 10 is stored in the storage unit 22. The CPU 21 reads out the information processing program 27 from the storage unit 22, loads the read-out program into the memory 23, and executes the loaded information processing program 27. The CPU 21 is an example of a processor of the present disclosure. As the information processing apparatus 10, for example, a personal computer, a server computer, a smartphone, a tablet terminal, a wearable terminal, or the like can be appropriately applied.

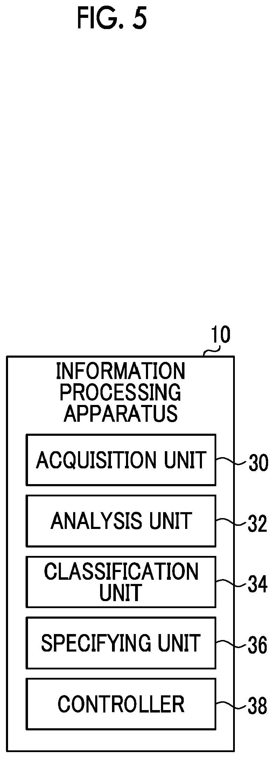

Next, with reference to Figs. 5 to 9, an example of a functional configuration of the information processing apparatus 10 according to the present embodiment will be described. As shown in Fig. 5, the information processing apparatus 10 includes an acquisition unit 30, an analysis unit 32, a classification unit 34, a specifying unit 36, and a controller 38. As the CPU 21 executes the information processing program 27, the CPU 21 functions as respective functional units of the acquisition unit 30, the analysis unit 32, the classification unit 34, the specifying unit 36, and the controller 38.

The acquisition unit 30 acquires a medical image including a plurality of lesion regions obtained by imaging a subject from the image server 5. Here, the medical image acquired by the acquisition unit 30 may consist of one image, or include a plurality of images, such as the medical image T consisting of the tomographic images T1 to Tm in Fig. 2. Thus, for example, one image may include a plurality of lesion regions, or each of a plurality of images may include a lesion region. Further, for example, in a case where a medical image includes a plurality of images, at least some of the images need only include the lesion region, and some of the images need not include the lesion region.

The analysis unit 32 extracts a region of interest from the medical image acquired by the acquisition unit 30. That is, the analysis unit 32 may extract at least a plurality of lesion regions from the medical image, and may also extract other regions of structures or the like. As a method for extracting the region of interest, a known method using a CAD technology, an AI technology, or the like can be appropriately applied. For example, the analysis unit 32 may extract a region of interest from a medical image by using a learning model such as a convolutional neural network (CNN) that has been trained to receive the medical image as an input and extract and output a region of interest included in the medical image.

In addition, the analysis unit 32 may generate a comment on findings regarding the extracted region of interest. As a method for generating a comment on findings, a known method using a CAD technology, an AI technology, or the like can be appropriately applied. For example, the analysis unit 32 may generate findings information of a region of interest by using a learning model such as a CNN that has been trained in advance to receive the region of interest extracted from the medical image as an input and output the findings information of the region of interest. After that, the analysis unit 32 may generate a comment on findings including the generated findings information. For example, the analysis unit 32 may generate a comment on findings by using a method using machine learning such as the recurrent neural network described in JP2019-153250A. Further, for example, the analysis unit 32 may generate a comment on findings by embedding findings information in a predetermined template.

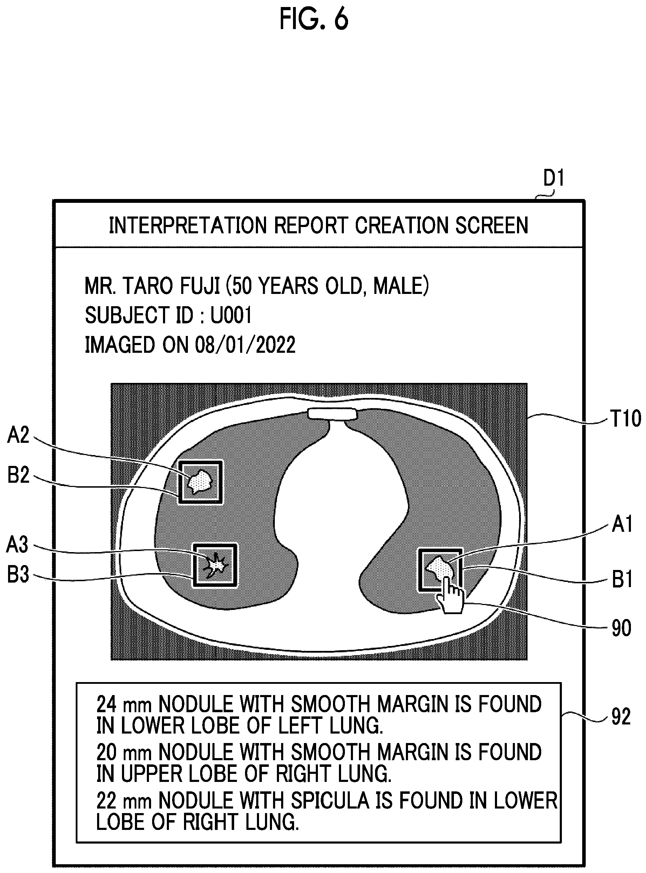

The controller 38 performs control to display the medical image on the display 24 by highlighting a plurality of lesion regions extracted by the analysis unit 32. Fig. 6 shows an example of a screen D1 displayed on the display 24 by the controller 38. The screen D1 includes a medical image T10, in which lesion regions A1 to A3 are emphasized by being surrounded by bounding boxes B1 to B3, respectively.

Note that a highlighting method is not limited to the method using the bounding boxes B1 to B3 as shown in Fig. 6. For example, the controller 38 may attach a marker such as an arrow to the vicinity of the lesion region, change a display method of colors and the like between the lesion region and other regions (so-called mask processing), or enlarge and display the portion of the lesion region in the medical image.

In addition, the controller 38 may perform control to display, on the display 24, a comment on findings generated by the analysis unit 32. The screen D1 of Fig. 6 includes comments on findings 92 regarding each of the lesion regions A1 to A3.

The classification unit 34 classifies a high or low degree of interest of at least one first lesion region among the plurality of lesion regions included in the medical image acquired by the acquisition unit 30. Specifically, the classification unit 34 may receive an input of a high or low degree of interest of the first lesion region. For example, the classification unit 34 may perform control to display, on the display 24, a screen for inputting the high or low degree of interest of the first lesion region.

For example, the classification unit 34 may first receive designation of the first lesion region for receiving the input of the degree of interest on the screen D1 of Fig. 6. A user, such as a radiologist, operates a mouse pointer 90 via the input unit 25 to designate a first lesion region into which the degree of interest is input. In the example shown in Fig. 6, the lesion region A1 is designated as the first lesion region.

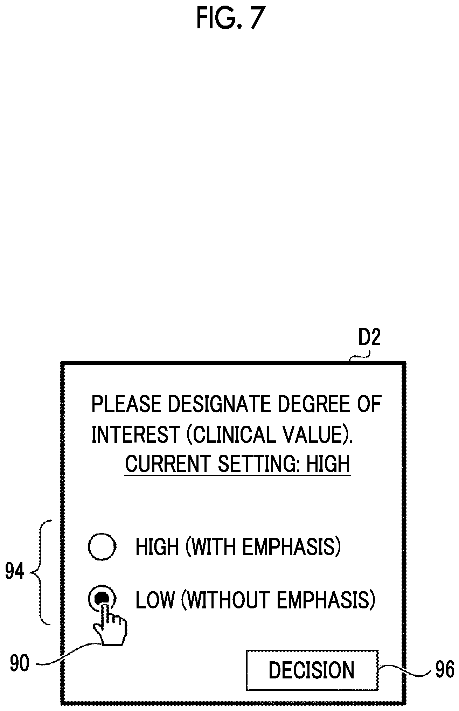

In a case where the first lesion region is designated by a user, the classification unit 34 performs control to display, on the display 24, a screen for receiving an input of a high or low degree of interest of the designated first lesion region. Fig. 7 shows an example of a screen D2 displayed on the display 24 by the classification unit 34. The screen D2 includes a radio button 94 for receiving an input of a high or low degree of interest, and a decision button 96. The user operates the mouse pointer 90 via the input unit 25, inputs the degree of interest using the radio button 94, and presses the decision button 96. The classification unit 34 classifies the high or low degree of interest of the first lesion region designated on the screen D1 based on the high or low degree of interest input on the screen D2. In the example shown in Fig. 6 and Fig. 7, the first lesion region A 1 is classified as having a low degree of interest.

In a case where the degree of interest of the first lesion region classified by the classification unit 34 is low, the specifying unit 36 specifies at least one second lesion region similar to the first lesion region among the plurality of lesion regions extracted by the analysis unit 32. Specifically, the specifying unit 36 may specify the second lesion region based on image features of the medical image. For example, the specifying unit 36 may extract an image feature amount of a medical image by using a learning model such as a CNN that has been trained in advance to receive the medical image as an input and output the feature amount of the input medical image. Then, the specifying unit 36 may derive a degree of similarity between the first lesion region and other lesion regions based on the image feature amount, and specify, as the second lesion region, a lesion region of which a similarity degree is equal to or higher than a predetermined threshold value. Note that, as the similarity degree, for example, the reciprocal of distances of feature amount vectors obtained by vectorizing the image feature amounts can be appropriately applied.

In addition, for example, the specifying unit 36 may specify, as the second lesion region, a lesion region similar to the first lesion region among the lesion regions in which a distance from the first lesion region is within a predetermined range. In other words, the specifying unit 36 may not specify, as the second lesion region, a lesion region in which the distance from the first lesion region is outside the predetermined range, even if the lesion region is similar to the first lesion region based on the image feature. Here, the distance from the first lesion region may be a distance within one medical image, or is a distance in a cranio-caudal direction in the case of a medical image consisting of a plurality of tomographic images as shown in Fig. 2.

In addition, for example, the specifying unit 36 may specify, as the second lesion region, a lesion region similar to the first lesion region among the lesion regions located in the same or related structure as the structure (organs, viscera, and tissues of the human body) in which the first lesion region is located. In other words, the specifying unit 36 may not specify, as the second lesion region, a lesion region located in another structure unrelated to the structure in which the first lesion region is located, even if the lesion region is similar to the first lesion region based on the image feature. For example, in a case where the first lesion region is located in the right kidney, the specifying unit 36 may specify, as the second lesion region, the lesion region similar to the first lesion region among the lesion regions located in the right kidney or the left kidney related to the right kidney. Note that the relationship between the structures may be stored in advance in, for example, the storage unit 22.

In addition, for example, the specifying unit 36 may specify the second lesion region by combining the conditions for the distance from the first lesion region and the structure in which the first lesion region is located. For example, the specifying unit 36 may specify, as the second lesion region, a lesion region similar to the first lesion region among the lesion regions in which the distance from the first lesion region is within the predetermined range and which are located in the same or related structure as the structure in which the first lesion region is located. For example, in a case where the first lesion region is located in the liver, the specifying unit 36 may not specify, as the second lesion region, the lesion region located in a structure unrelated to the liver, such as the lung and the diaphragm even if the distance from the first lesion region is short.

The controller 38 performs control to display, on the display 24, the display method of the first lesion region classified as having a low degree of interest by the classification unit 34 and the second lesion region specified by the specifying unit 36 in a manner different from that of the other lesion regions. Specifically, the controller 38 performs control to display, on the display 24, the medical image such that the first lesion region classified as having a low degree of interest by the classification unit 34 and the second lesion region specified by the specifying unit 36 are not emphasized more than the other lesion regions.

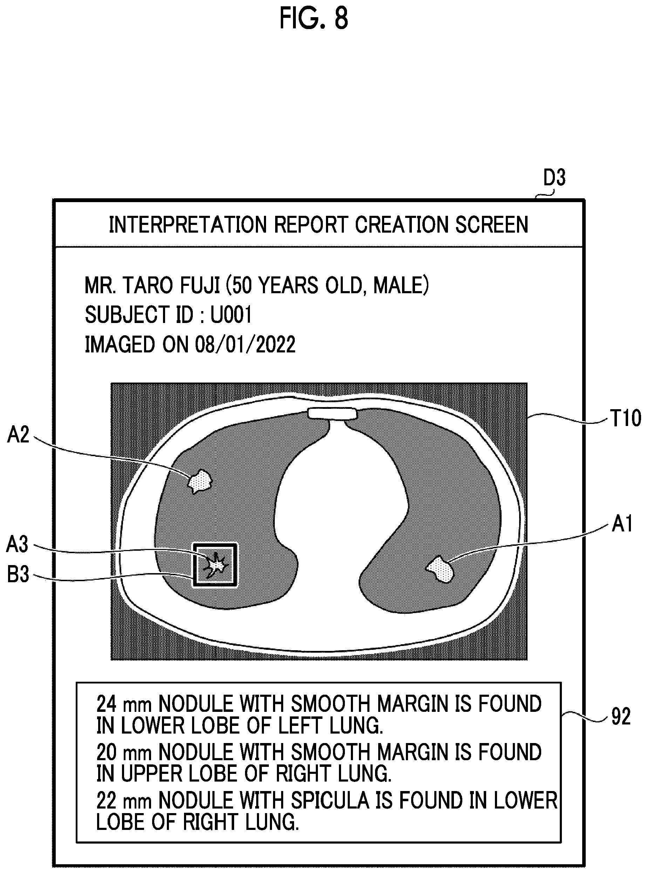

Fig. 8 shows an example of a screen D3 displayed on the display 24 by the controller 38. The screen D3 is a screen transitioned from the screen D1 in a case where the lesion region A2 is specified as the second lesion region similar to the first lesion region A1 after the input that the degree of interest of the first lesion region A1 is low on the screen D2. On the screen D3, the bounding boxes B1 and B2 surrounding the first lesion region A1 and the second lesion region A2 on the screen D1 are removed. On the other hand, the lesion region A3 which was not specified as the second lesion region similar to the first lesion region A1 (that is, specified not to be similar to the first lesion region A1) is highlighted by the bounding box B3 continuously from the screen D1.

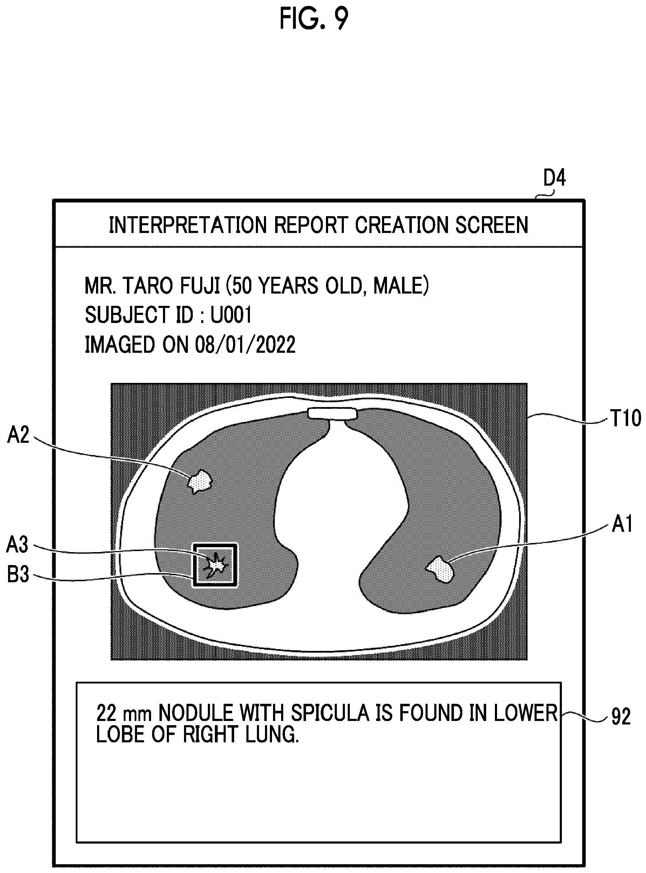

In addition, the controller 38 may delete the portions related to the first lesion region and the second lesion region classified as having a low degree of interest from the comment on findings generated by the analysis unit 32. Fig. 9 shows an example of a screen D4 displayed on the display 24 by the controller 38 in this case. From the comment on findings 92 on the screen D4, the portions related to the first lesion region A1 and the second lesion region A2 are deleted. On the other hand, the lesion region A3 which was not specified as the second lesion region similar to the first lesion region A1 (that is, specified not to be similar to the first lesion region A1) is included in the comment on findings 92 continuously from the screen D1.

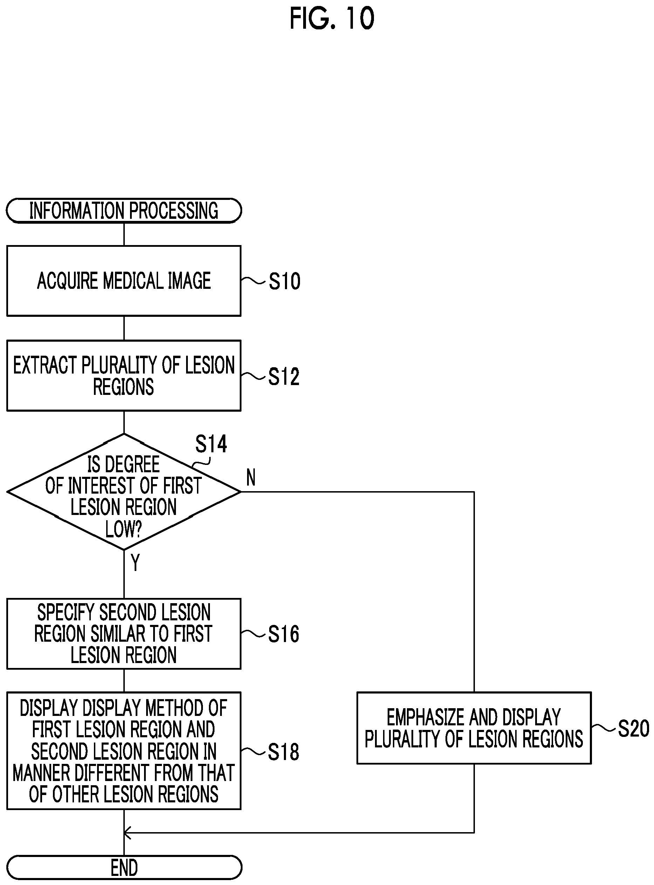

Next, with reference to Fig. 10, operations of the information processing apparatus 10 according to the present embodiment will be described. In the information processing apparatus 10, as the CPU 21 executes the information processing program 27, information processing shown in Fig. 10 is executed. The information processing is executed, for example, in a case where the user gives an instruction to start execution via the input unit 25.

In Step S10, the acquisition unit 30 acquires a medical image including a plurality of lesion regions obtained by imaging a subject from the image server 5. In Step S12, the analysis unit 32 extracts a plurality of lesion regions from the medical image acquired in Step S10. In Step S14, the classification unit 34 classifies a high or low degree of interest of at least one first lesion region among the plurality of lesion regions extracted in Step S12.

In a case where it is classified in Step S14 that the degree of interest of the first lesion region is low (Step S14 is Y), the process proceeds to Step S16. In Step S16, the specifying unit 36 specifies at least one second lesion region similar to the first lesion region among the plurality of lesion regions extracted in Step S12. In Step S18, in a case where the plurality of lesion regions extracted in Step S12 are emphasized and displayed on the display 24, the controller 38 performs control to display, on the display 24, the display method of the first lesion region and the second lesion region specified in Step S16 in a manner different from that of the other lesion regions, and ends this information processing.

On the other hand, in a case where it is classified in Step S14 that the degree of interest of the first lesion region is high (Step S14 is N), the process proceeds to Step S20. In Step S20, the controller 38 performs control to emphasize and display, on the display 24, the plurality of lesion regions extracted in Step S12, and ends this information processing.

As described above, the information processing apparatus 10 according to one aspect of the present disclosure comprises at least one processor, and the processor is configured to: acquire a medical image including a plurality of lesion regions; classify a high or low degree of interest of at least one first lesion region among the plurality of lesion regions; specify at least one second lesion region similar to the first lesion region among the plurality of lesion regions in a case where the degree of interest is low; and make a display method of the first lesion region and the second lesion region classified as having a low degree of interest different from a display method of other lesion regions in a case where the plurality of lesion regions are highlighted and the medical image is displayed on the display 24.

That is, with the information processing apparatus 10 according to the present embodiment, since the display method can be made different between a lesion having a high degree of interest and a lesion having a low degree of interest in the interpretation of the medical image, complication can be suppressed and visibility can be improved. In addition, by utilizing the degree of interest of similar lesions, it is possible to more efficiently change the display method between the lesion having a high degree of interest and the lesion having a low degree of interest, thereby supporting the interpretation of a medical image.

In addition, in the above-described embodiment, regarding the degree of interest of the first lesion region, the form of receiving the input of high or low degree of interest by the user has been described, but the present disclosure is not limited thereto. For example, the classification unit 34 may acquire a predetermined degree of interest for the lesion region similar to the first lesion region as the degree of interest of the first lesion region. As described above, the report DB 8 may store a high or low degree of interest determined for the lesion region in the interpretation work. Therefore, the classification unit 34 may refer to the report DB 8 and use the degree of interest determined at the time of past interpretation of the lesion region similar to the first lesion region as the degree of interest of the first lesion region.

Here, the lesion region similar to the first lesion region may be included in a medical image obtained by imaging the same subject as the subject whose image is currently being interpreted or may be included in a medical image obtained by imaging a different subject. For example, in a case of performing follow-up observation on the same subject, the degree of interest of the same lesion region input in the past interpretation work may be used. Further, for example, regardless of whether the subjects are the same or different, the degree of interest is determined for each type of lesion region according to the policies of the radiologist, the medical institution, and the like, and the degree of interest of other lesion regions of the same type may be used.

Further, for example, the classification unit 34 may estimate a degree of interest of the first lesion region based on whether or not there is a description of a lesion region similar to the first lesion region in an interpretation report describing another medical image including the lesion region. Specifically, the classification unit 34 refers to the report DB 8 and searches for interpretation reports describing other medical images including a lesion region similar to the first lesion region. In addition, the classification unit 34 may estimate that the degree of interest of the first lesion region is low in a case where the interpretation report obtained by the search does not include a description of the lesion region similar to the first lesion region. On the other hand, the classification unit 34 may estimate that the degree of interest of the first lesion region is high in a case where the interpretation report obtained by the search includes a description of the lesion region similar to the first lesion region.

In addition, in the above-described embodiment, as shown in Fig. 6, first, a form in which all the lesion regions are highlighted has been described, but the present disclosure is not limited thereto. As in each of the above examples, in a case where a predetermined degree of interest is acquired for the degree of interest of the first lesion region, or in a case where estimation is performed based on the description of another interpretation report, the first lesion region and the second lesion region having a low degree of interest may not be highlighted from the beginning.

In addition, in the description with reference to Figs. 6 to 8 of the above-described embodiment, the form in which the first lesion region and the second lesion region are included in one medical image T10 has been described, but the present disclosure is not limited thereto. As described above, the medical image acquired by the acquisition unit 30 may include a plurality of images, such as the medical image T consisting of the tomographic images T1 to Tm in Fig. 2. Thus, for example, after the classification unit 34 classifies the first lesion region included in a certain tomographic image as having a low degree of interest, the specifying unit 36 may specify the second lesion region included in another tomographic image.

In addition, in the description with reference to Figs. 6 to 8 of the above-described embodiment, a form of specifying one second lesion region A2 similar to the first lesion region A1 classified as having a low degree of interest has been described, but the present disclosure is not limited thereto. For example, the specifying unit 36 may specify a plurality of second lesion regions similar to the first lesion region classified as having a low degree of interest.

In addition, in the description with reference to Figs. 6 to 8 of the above-described embodiment, the form of classifying a high or low degree of interest of one first lesion region A1 has been described, but the present disclosure is not limited thereto. For example, the classification unit 34 may classify the high or low degree of interest of each of the plurality of first lesion regions. The specifying unit 36 may specify at least one second lesion region similar to each of the first lesion regions classified as having a low degree of interest.

In addition, in the above-described embodiment, the form in which the analysis unit 32 generates findings information and a comment on findings based on the medical image has been described, but the present disclosure is not limited thereto. For example, the analysis unit 32 may acquire findings information and comments on findings stored in advance in the storage unit 22, the image server 5, the image DB 6, the report server 7, the report DB 8, and other external devices. Alternatively, for example, the analysis unit 32 may acquire findings information and comments on findings manually input by the user via the input unit 25.

In the above embodiment, for example, as hardware structures of processing units that execute various kinds of processing, such as the acquisition unit 30, the analysis unit 32, the classification unit 34, the specifying unit 36, and the controller 38, various processors shown below can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured by one of the various processors, or may be configured by a combination of the same or different kinds of two or more processors (for example, a combination of a plurality of FPGAs or a combination of the CPU and the FPGA). In addition, a plurality of processing units may be configured by one processor.

As an example in which a plurality of processing units are configured by one processor, first, there is a form in which one processor is configured by a combination of one or more CPUs and software as typified by a computer, such as a client or a server, and this processor functions as a plurality of processing units. Second, as represented by a system on chip (SoC) or the like, there is a form of using a processor for realizing the function of the entire system including a plurality of processing units with one integrated circuit (IC) chip. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

In the above embodiment, the information processing program 27 is described as being stored (installed) in the storage unit 22 in advance; however, the present disclosure is not limited thereto. The information processing program 27 may be provided in a form recorded in a recording medium such as a compact disc read only memory (CD-ROM), a digital versatile disc read only memory (DVD-ROM), and a universal serial bus (USB) memory. In addition, the information processing program 27 may be downloaded from an external device via a network. Further, the technology of the present disclosure extends to a storage medium for storing the information processing program non-transitorily in addition to the information processing program.

The technology of the present disclosure can be appropriately combined with the above-described embodiment and examples. The described contents and illustrated contents shown above are detailed descriptions of the parts related to the technology of the present disclosure, and are merely an example of the technology of the present disclosure. For example, the above description of the configuration, function, operation, and effect is an example of the configuration, function, operation, and effect of the parts according to the technology of the present disclosure. Therefore, needless to say, unnecessary parts may be deleted, new elements may be added, or replacements may be made to the described contents and illustrated contents shown above within a range that does not deviate from the gist of the technology of the present disclosure.

### Explanation of References

1: information processing system
2: imaging apparatus
3: interpretation WS
4: medical care WS
5: image server
6: image DB
7: report server
8: report DB
9: network
10: information processing apparatus
21: CPU
22: storage unit
23: memory
24: display
25: input unit
26: network I/F
27: information processing program
28: bus
30: acquisition unit
32: analysis unit
34: classification unit
36: specifying unit
38: controller
90: mouse pointer
92: comment on findings
94: radio button
96: decision button
AA, A1 to A3: lesion region
B1 to B3: bounding box
D1 to D4: screen
SA: region of structure
T, T10: medical image
T1 to Tm, Tx: tomographic image

## Claims

1. An information processing apparatus comprising at least one processor, wherein the processor is configured to:
acquire a medical image including a plurality of lesion regions;
classify a high or low degree of interest of at least one first lesion region among the plurality of lesion regions;
specify at least one second lesion region similar to the first lesion region among the plurality of lesion regions in a case where the degree of interest is low; and
make a display method of the first lesion region and the second lesion region classified as having a low degree of interest different from a display method of other lesion regions in a case where the plurality of lesion regions are highlighted and the medical image is displayed on a display.

2. The information processing apparatus according to claim 1, wherein the processor is configured to display the medical image on the display such that the first lesion region and the second lesion region classified as having a low degree of interest are not emphasized more than the other lesion regions.

3. The information processing apparatus according to claim 1, wherein the processor is configured to specify the second lesion region based on an image feature of the medical image.

4. The information processing apparatus according to claim 1, wherein the processor is configured to receive an input of the high or low degree of interest.

5. The information processing apparatus according to claim 1, wherein the processor is configured to acquire a predetermined degree of interest for a lesion region similar to the first lesion region as a degree of interest of the first lesion region.

6. The information processing apparatus according to claim 1, wherein the processor is configured to estimate a degree of interest of the first lesion region based on whether or not there is a description of a lesion region similar to the first lesion region in an interpretation report describing another medical image including the lesion region.

7. The information processing apparatus according to claim 6, wherein the processor is configured to estimate that a degree of interest of the first lesion region is low in a case where there is no description of the lesion region similar to the first lesion region in the interpretation report describing another medical image including the lesion region.

8. The information processing apparatus according to claim 1, wherein the processor is configured to specify, as the second lesion region, a lesion region similar to the first lesion region among lesion regions in which a distance from the first lesion region is within a predetermined range.

9. An information processing method comprising:
acquiring a medical image including a plurality of lesion regions;
classifying a high or low degree of interest of at least one first lesion region among the plurality of lesion regions;
specifying at least one second lesion region similar to the first lesion region among the plurality of lesion regions in a case where the degree of interest is low; and
making a display method of the first lesion region and the second lesion region classified as having a low degree of interest different from a display method of other lesion regions in a case where the plurality of lesion regions are highlighted and the medical image is displayed on a display.

10. A computer-readable storage medium storing an information processing program for causing a computer to execute a process comprising:
acquiring a medical image including a plurality of lesion regions;
classifying a high or low degree of interest of at least one first lesion region among the plurality of lesion regions;
specifying at least one second lesion region similar to the first lesion region among the plurality of lesion regions in a case where the degree of interest is low; and
making a display method of the first lesion region and the second lesion region classified as having a low degree of interest different from a display method of other lesion regions in a case where the plurality of lesion regions are highlighted and the medical image is displayed on a display.
